# EUROPÄISCHE PATENTANMELDUNG

(11) **EP 2 116 233 A1**
(43) Veröffentlichungstag der Anmeldung: **11.11.2009**
(21) Anmeldenummer: 09006134.2
(22) Anmeldetag: 05.05.2009
(51) Int. Cl.: A61K 9/70, A61K 9/06, A61P 17/02, A61K 47/36, A61L 27/20

(54) **Zweikomponenten-Alginat**

(30) Priorität: 05.05.2008 DE 102008022086
(71) Anmelder: Schulz, Hans, H., 51379 Leverkusen (DE)
(72) Erfinder: Schulz, Hans, H., 51379 Leverkusen (DE)
(74) Vertreter: Andrae, Steffen

(57) **Zusammenfassung**

Alginatmassen, erhältlich durch Vermischen einer ersten alginatfreien Komponente A, enthaltend Calciumionenspender, Hilfsstoffe und nicht wässrige, aber wassermischbare Lösungsmittel, mit einer zweiten Komponente B, bestehend aus einer Lösung von löslichen Alginaten in Wasser-Glycerin-Gemischen, insbesondere zur Verwendung als Wundauflagen z.B. im Mundbereich (in der Dentalmedizin) und für oberflächliche Wunden, z.B. für dermatologische Anwendungen.

## Beschreibung

Die Erfindung betrifft eine Alginatmasse, die aus zwei Komponenten hergestellt wird, wobei das Alginat und der Calciumionenspender in getrennten Komponenten vorliegen, die durch Vermischen zur Reaktion gebracht werden, und die Verwendung dieser Alginatmasse als Wundauflage in der Human-, Zahn- und Veterinärmedizin.

Alginatmassen sind als härtende, dentale Abformmassen bekannt und in DE 35 11 721, EP 0 359 189 oder EP 0 534 264 beschrieben. Zur Herstellung dieser Massen werden lösliche Alginatsalze (Natrium-, Kalium-, Ammonium- oder Triethanolamin-Salze), Calciumsalze, Phosphate, Hilfsstoffe und ggf. nichtwässrige Lösungsmittel in einer und mit Gelbildnern verdicktes Wasser in einer anderen Komponente zur Verfügung gestellt und durch Mischen zur Vernetzung zu Alginatmassen gebracht. Nachteilig bei dieser Vorgehensweise ist, dass es sich nicht um ein echtes Zweikomponentensystem handelt, denn die hauptsächlich reagierenden Komponenten, das Alginat und die vernetzenden Calciumionen sind in einer Komponente untergebracht und werden durch das Wasser in der anderen Komponente nur aktiviert. Dementsprechend ist die Alginat- und Calciumionen enthaltende Komponente potentiell instabil, denn durch Feuchtigkeit kommt es zu einer Vorreaktion. Dadurch ist die Stabilität dieser Komponente begrenzt.

Wünschenswert wären daher echte Zweikomponentensysteme, bei denen Alginat und Calciumspender in getrennten Komponenten vorliegen.

Die WO 2008/000466 beschreibt Wundauflagen aus Alginatmassen, bei denen ebenfalls Alginat und Calciumionenspender in einer Komponente untergebracht sind. Auch hier existiert das Stabilitätsproblem. Auch die Elastizität, besonders nach Wasserquellung, und die Wasseraufnahme der resultierenden Wundauflagen sind für einige Anwendungen nicht ganz befriedigend.

Wünschenswert sind daher Wundauflagen aus Alginatmassen mit besseren Eigenschaften und stabileren Ausgangskomponenten. Sie sollen vor allem in situ härtbar sein und sich dadurch passgenau der Wundoberfläche anpassen und die Funktion einer schützenden Wundabdeckung und eines Wirkstoffdepots übernehmen.

Es wurde nun gefunden, dass durch Vermischen einer Komponente, bestehend aus der Lösung von Alginaten in Wasser-Glycerin-Gemischen und einer anderen Komponente enthaltend Calciumionenspender, Hilfsstoffe und nichtwässrige, aber mit Wasser mischbare Lösungsmittel, elastische, mit Wasser gut quellbare Alginatmassen entstehen, die auch noch im gequollenen Zustand eine gute Festigkeit aufweisen. Sie sind für alle unter der WO 2008/000466 beschriebenen Anwendungen und mit den dort beschriebenen Applikationsmethoden vorteilhaft anwendbar und zeichnen sich durch eine höhere Wasseraufnahme, eine bessere Homogenität und eine höhere Festigkeit, besonders im gequollenen Zustand aus.

Gegenstand der Erfindung sind daher Alginatmassen, erhältlich durch Vermischen
einer Komponente A, bestehend aus
Calciumionenspendern, Hilfsstoffen und nicht wässrigen, aber wassermischbaren Lösungsmitteln, und
einer Komponente B, bestehend aus
einer Lösung von löslichen Alginaten in Wasser-Glycerin-Gemischen.

Ein weiterer Gegenstand der Erfindung ist die Verwendung der erfindungsgemäßen Alginatmassen als Wundauflage.

Lösliche Alginate im Sinne der Erfindung sind ihre Natrium-, Kalium-, Ammonium- oder Aminsalze, besonders bevorzugt das Triethanolaminsalz. Sie werden in Konzentrationen von 2 bis 8 Gew.-% in einer Mischung aus 2 bis 10 Gew.-% Glycerin und 90 bis 98 Gew.-% Wasser gelöst. Der Glycerinanteil im wässrigen Lösungsmittel erhöht dabei die Löslichkeit des Alginats.

Calciumspender im Sinne der Erfindung sind Calciumsalze mit einer Löslichkeit von unter zwei Gew.-% in Wasser, wie Calciumsulfate in verschiedenem Hydratisierungsgrad, vorzugsweise als Semi- oder Dihydrat, oder Calciumsilikat. Ihr Anteil an der Komponente A beträgt 10 bis 20 Gew.-%.

Nicht wässige, aber mit Wasser mischbare Lösungsmittel sind Polyethylenglykole mit einer Molmasse von 200-600 g/mol und Glycerin. Ihr Anteil an der Komponente A beträgt 50 bis 65 Gew.-%.

In der Komponente A werden verschiedene Hilfsstoffe, z.T. obligatorisch, z.T. optional, eingesetzt:

0,1 bis 3 Gew.-% Verzögerer, wie Natrium-, Kalium-, Ammoniumphosphat und die entsprechenden Mono- oder Dihydrogenphosphate oder Pyrophosphate.

3 bis 7 Gew.-% Metalloxide, wie Magnesium oder Zinkoxid.

15 bis 25 Gew.-% wasserlösliche Polymere außer Alginaten, wie z.B. Carboxymethylcellulose (als Na-Salz), Hydroxyethylcellulose, Polyacrylsäure (als Na-Salz), Polyvinylalkohol, vorzugsweise Carboxymethylcellulose Na-Salz.

0-2 Gew.-% Wirkstoffe, wie Biguanide, wie z.B. Chlorhexidin, Alexidin oder Polyhexanid, wie Formaldehyd abgebende Stoffe, die das Strukturelement Z-CH2-Z enthalten, wobei Z für ein Heteroatom, vorzugsweise Stickstoff steht, das beliebige Substituenten tragen kann oder Teil eines heterocyclischen Rings sein kann, wie z.B. Taurolidin, wie quaternisierte Stickstoffheterocyclen, wie z.B. Octenidindihydrochlorid oder Cetylpyridiniumbromid, wie quartäre Ammoniumionen, wie z.B. Undecylenamidopropyl Betain, oder wie Gyrase-Hemmer, die in EP 1 244 434 beschrieben sind, wie Moxifloxazin oder Gatifloxazin.

0,1 bis 5 Gew.-% rheologische Hilfmittel, die die Komponente A verdicken, um die Sedimentation der anorganischen Bestandteile zu vermindern. Hierbei sind Polyurethanverdicker (sog. Assoziativverdicker) besonders bevorzugt, die z.B. von der Fa. Borchers unter dem Namen Borchigel erhältlich sind.

Die Komponenten A und B werden im Volumenverhältnis 1:3 bis 1:10 gemischt, vorzugsweise im Verhältnis 1:4 bis 1:8.

Die Mischung kann händisch erfolgend, vorzugsweise aber durch einen Statikmischer. Dabei werden die Komponenten in Doppelkammerkartuschen aufbewahrt und mit einer Doppelkammerspritze durch einen vorgesetzten Statikmischer gedrückt. Die Härtung zu einer elastischen Alginatmasse erfolgt in einer bis 5 Minuten. Die Masse kann direkt auf Wunden aufgetragen werden, wobei passgenau an die Wundoberfläche angepasste Wundauflagen resultieren. Auch für kosmetische Anwendungen durch einen Fachmann ist diese Vorgehensweise geeignet.

### Beispiele

### Beispiel 1

### Herstellung der Komponente A

Man vermischte unter hoher Scherung 6,83g Calciumsulfat Hemihydrat, 6,83g Calciumsulfat Dihydrat, 2,01g Natriumpyrophosphat, 4,02g Magnesiumoxid, 19,88g Carboxymethylcellulose (CMC Sodium Low Viscosity, Viskosität 500-2500 mPas (4% in Wasser, 25 °C)), Sigma-Aldrich Chemie GmbH, Steinheim, Deutschland), 28,72g PEG 400, 29,72g Glycerin, 0,6g Polyhexanid (Vantocil 100, Arch Biocides, Manchester, GB) und 2g Borchigel L75N (Borchers).

### Herstellung der Komponente B

Man vermischte unter Rühren 5g Triethanolamin-Alginat (Protanal TA250M der Fa.BioPolymer, Brüssel, Belgien), 5g Glycerin und 90g Wasser.

Man befüllte eine Doppelkartusche in einem Mischungsverhältnis 1:5 mit den Komponenten A und B, drückte die Komponenten durch einen Statikmischer und erhielt eine elastische, transluzente Alginatmasse, die währende 8h Wasserlagerung 110 Gew.-% Wasser aufnahm. Die Transluzenz erlaubt die Beobachtung des Heilungsfortschritts bei der Anwendung als Wundauflage.

### Vergleichsbeispiel

Man stellte die Komponenten A und B wie in Beispiel 2 der WO 2008/000466 her und mischte sie im Verhältnis 1:5, wie in Beispiel 1 beschrieben. Man erhielt eine opake Alginatmasse, die während 8h Wasserlagerung 75 Gew.-% Wasser aufnahm. Die gequollene Alginatmasse ließ sich zwischen den Fingern zerdrücken, was bei der Alginatmasse nach Beispiel 1 nach Wasserlagerung nicht der Fall war.

## Patentansprüche

1. Alginatmassen, erhältlich durch Vermischen einer Komponente A, bestehend aus Calciumionenspendern, Hilfsstoffen und nicht wässrigen, aber wassermischbaren Lösungsmitteln und einer Komponente B, bestehend aus einer Lösung von löslichen Alginaten in Wasser-Glycerin-Gemischen.

2. Alginatmassen nach Anspruch 1, wobei die Komponente A 0,1 bis 5 Gew.-% rheologische Hilfsmittel enthält.

3. Alginatmassen nach Anspruch 2, wobei als rheologische Hilsmittel Polyurethanverdicker eingesetzt werden.

4. Alginatmassen nach Anspruch 1, **dadurch gekennzeichnet, dass** die Komponente A kein Alginat enthält.

5. Verwendung der Alginatmassen nach Anspruch 1 bis 4 als Wundauflagen.
